(19)

Europäisches
Patentamt

European
Patent Office

Office européen
des brevets

(11) **EP 2 351 562 A1**

(12) **EUROPEAN PATENT APPLICATION**
published in accordance with Art. 153(4) EPC

(43) Date of publication:
**03.08.2011 Bulletin 2011/31**

(21) Application number: **09823281.2**

(22) Date of filing: **26.10.2009**

(51) Int Cl.:
***A61K 31/198*** *(2006.01)*   ***A61K 9/14*** *(2006.01)*
***A61P 3/00*** *(2006.01)*   ***A61P 3/02*** *(2006.01)*
***A61P 7/00*** *(2006.01)*   ***A61P 21/00*** *(2006.01)*
***A61P 43/00*** *(2006.01)*

(86) International application number:
**PCT/JP2009/005626**

(87) International publication number:
**WO 2010/050168 (06.05.2010 Gazette 2010/18)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **27.10.2008 JP 2008275367**

(71) Applicant: **Ajinomoto Co., Inc.
Tokyo 104-8315 (JP)**

(72) Inventors:
  • **KOYAMA, Masako
    Kawasaki-shi
    Kanagawa 210-8681 (JP)**

  • **KIMURA, Momoko
    Kawasaki-shi
    Kanagawa 210-8681 (JP)**
  • **TANEGAWA, Tetsuo
    Saga-shi
    Saga 840-2193 (JP)**

(74) Representative: **Nicholls, Kathryn Margaret et al
Mewburn Ellis LLP
33 Gutter Lane
London
EC2V 8AS (GB)**

(54) **SOLID SOLUTION OF VALINE, ISOLEUCINE AND LEUCINE, AND METHOD FOR PRODUCING SAME**

(57)    [Problem] A valine, isoleucine, leucine mixture wich is excellent in the reduction of bitter taste and improved dissolution speed is provided.

[Solving Means] The above problem is solved by a solid solution composed of three amino acids of valine, isoleucine, leucine, and a solid solution composed of these and at least one of acid selected from the group consisting of neutral aliphatic amino acid, neutral hydroxy amino acid, neutral acid amide amino acid, acidic amino acid, basic amino acid, hydroxy acid.

EP 2 351 562 A1

**Description**

Technical Field

This invention relates to a solid solution of amino acids, and more particularly, relates to a solid solution composed of three amino acids of valine, isoleucine, leucine and a solid solution composed of the three amino acids and further an amino acid which is at least one type selected from the group consisting of neutral aliphatic amino acid, neutral hydroxy amino acid, neutral acid amide amino acid, acidic amino acid, basic amino acid, hydroxy acid.

Background Art

Amino acids are constituents of proteins which are indispensable for maintaining health, and are widely used for foods, supplements, medicines and the like. Valine, isoleucine, leucine which are branched chain amino acids (BCAA), are particularly suitable for compersating nitrogen metabolism while exercising, and marketed as solid foods wherein valine, isoleucine, leucine have been mixed, foods which are blended in the state of powder and are to be dissolved in water and eaten, and drinking water, for the purpose of suppressing damage of muscles during hard exercising for a long period and of recovering fatigue rapidly. Moreover, a mixture of valine, isoleucine, leucine is used as oral solid medicine which improves easy fatigue and accumulation of ascites of cirrhotics.

On the other hand, valine, isoleucine, leucine are known to have bitter taste, and particularly, isoleucine exhibits bitter tastes from lower concentration having a stimulus threshold of 90mg/dl (Non-Patent Document 1).

As a method of improving taste of substances having bitter taste, there are a method of reducing bitter taste by using a masking flavor, or by taking a balance with other taste, such as sweet taste, acid taste, a method of coating the surface of tablets so as to take them without feeling the bitter taste, and the like. Particularly, in the case of easily eatable solid foods and solid medicines of which melting in the mouth is taken into consideration, not the coating method but the method of controlling taste using acid taste, sweet taste, flavor is employed.

Incidentally, these amino acids are manufactured separately. That is, in the manufacture of respective amino acids, since other amino acids are impurities, they are heretofore removed by crystallization to recover crystals, operation of ion exchange resin, or the like. However, solid solution composed of two kinds, i.e. valine and isoleucine, and solid solution composed of two kinds, i.e. leucine and valine, are published in papers (Non-Patent Document 2, Non-Patent Document 3).

The solubility at 20°C of valine, isoleucine, leucine is 5.75g, 4.02g, 2.38g, and therefore, they are water-soluble (Non-Patent Document 1). However, since the side chains of their amino acids are carbon chains being hydrophobic, phenomena, such as floating on water surface occur. Accordingly, when preparing an aqueous solution, it is necessary to be stirred well and heated. In the patent concerning the solid solution composed of three amino acids of valine, isoleucine, leucine which was applied previously, it is described that solubility of the solid solution is improved compared with a powder mixture of single amino acids.

In addition, pulverized BCAA are occasionally used in order to improve solubility. However, valine, isoleucine, leucine have a lower limit concentration of explosion in the range of 20-30mg/l, and risk of dust explosion is very high. Accordingly, this is not suitable for mass operation.

[Non-Patent Document 1] Ed. by Ajinomoto Co., Inc. "Amino Acid Handbook", page 47, Kogyo Chosa kai, April 1, 2003
[Non-Patent Document 2] Ind. Eng. Chem. Res., vol. 44, pages 3284-3288, 2005
[Non-Patent Document] Fluid Phase Equilibria, vol. 224, pages 245-249, 2004

Disclosure of the Invention

Problems to be Solved by the Invention

In order to solve the problems of bitter taste of valine, isoleucine, leucine, when masking flavor, acidulant or sweetening is used, their use and blending are restricted by the flavor or taste. Moreover, cost increases, and further, for the medical use, they render medicines bulky to degrade drinkability.

The crystals of valine, isoleucine, leucine require a long time for dissolution due to water repellency, and have a problem in operating ability. For this problem, there is a solution to pulverize the crystals under the present conditions. However, yield is lowered by fly loss, and moreover, there is a risk of dust explosion.

For beverages to be dissolved in water upon drinking, such as powdered beverages, when the beverage containing valine, isoleucine, leucine is prescribed a quantity to be drunk, the beverage is to be drunk before dissolved off. When it is considered important that the appearance to be dissolved completely, blending amounts of valine, isoleucine, leucine are restricted. For soft drinks blended with amino acids, a dissolution with heating process of valine, isoleucine, leucine takes a long time for dissolving them completely upon manufacture, and rapid dissolution of them is required for efficient manufacture. Even in the case of solid foods and solid medicines which are eaten as they are without being dissolved in water, it is favorable to have rapid dissolution, because melting ability in the mouth is a favorable touch in the mouth.

Although solid solutions of two kinds, i.e. valine and isoleucine or valine and leucine are known, they are academic reports of crystalline states, and taste upon oral intake has not been considered at all. Moreover, the papers concern to obtain solid solutions from solutions, and there is no description concerning dissolution speed of the obtained solid solutions into water.

It is an object of the invention to provide a mixture of valine, isoleucine, leucine having excellent reduction of bitter taste and improved dissolution speed.

It is another object of the invention to provide a mixture of valine, isoleucine, leucine having remarkably improved dissolution speed.

Means for Solving Problems

In order to achieve the above objects, the inventors investigated, and found that, by conducting crystallization from a mixture solution of dissolved valine, isoleucine, leucine, these can be obtained in a form of solid solution, and the solid solution solves the aforementioned objects.

And, they further found that, by conducting crystallization from a mixture solution of dissolved valine, isoleucine, leucine and at least one type of acid selected from the group consisting of neutral aliphatic amino acid, neutral hydroxy amino acid, neutral acid amide amino acid, acidic amino acid, basic amino acid, hydroxy acid, these can be obtained in a form of solid solution, and the solid solution also solves the aforementioned objects to reach the present invention. Thus, the present invention relates to;

(1) A solid solution composed of three amino acids of valine, isoleucine, leucine.

(2) A solid solution as set forth in claim 1, having a composition ratio of 5-85 w/w % of valine, 3-80 w/w % of isoleucine, 3-80 w/w % of leucine.

(3) A solid solution as set forth in claim 1 or 2, wherein a powder X-ray diffraction pattern indicates main peaks at a diffraction angle (2θ) of 6-7, 12-14 (unit:degree).

(4) A solid solution composed of valine, isoleucine, leucine and at least one of acid selected from the group consisting of neutral aliphatic amino acid, neutral hydroxy amino acid, neutral acid amide amino acid, acidic amino acid, basic amino acid, hydroxy acid.

(5) A solid solution as set forth in claim 4, wherein the aliphatic amino acid is glycine, alanine, norleucine, homoleucine.

(6) A solid solution as set forth in claim 4, wherein the neutral hydroxy amino acid is serine, threonine.

(7) A solic solution as set forth in claim 4, wherein the neutral acid amide amino acid is asparagines monohydrate, glutamine.

(8) A solid solution as set forth in claim 4, wherein the acidic amino acid is glutamic acid, aspartic acid.

(9) A solid solution as set forth in claim 4, wherein the basic amino acid is arginine, lysine, histidine.

(10) A solid solution as set forth in claim 4, wherein the hydroxy acid is malic acid, citric acid, tartaric acid.

(11) A solid solution as set forth in any one of claims 5-10, which has a composition ratio of 5-85 w/w % of valine, 3-80 w/w % of isoleucine, 3-80 w/w % of leucine, 0.01-50 w/w % of at least one of acid selected from the group consisting of neutral aliphatic amino acid, neutral hydroxy amino acid, neutral acid amide amino acid, acidic amino acid, basic amino acid, hydroxy acid.

(12) A solid solution as set forth in any one of claims 5-11, wherein a powder X-ray diffraction pattern indicates the strongest peak at a diffraction angle (2θ) of 6-7°, and the first, second strongest peaks existing at the position shown below derived from at least one of acid selected from the group consisting of neutral aliphatic amino acid, neutral hydroxy amino acid, neutral acid amide amino acid, acidic amino acid, basic amino acid, hydroxy acid, are not detected, or detected as smaller peaks than those of a powder mixture having the same composition:

Glycine diffraction angle (2θ) = 29.8°, 23.9°, alanine diffraction angle (2θ) = 20.6°, 28.9°, threonine diffraction angle (2θ) = 35.0°, 28.5°, glutamine diffraction angle (2θ) = 23.4°, 25.0°, asparagine monohydrate diffraction angle (2 θ) = 29.2°, 18.1°, glutamic acid diffraction angle (2θ) = 10.3°, 20.5°, aspartic acid diffraction angle (2θ) = 23.8°, 11.9°, arginine diffraction angle (2θ) = 18.3°, 27.4°, citric acid diffraction angle (2θ) = 15.2°, 18.2°, malic acid diffraction angle (2θ) = 20.1°, 23.5°, tartaric acid diffraction angle (2θ) = 20.7°, 35.8°.

(13) A solid solution as set forth in any one of claims 1-12, having a mean particle size of 50-1000 μ m.

(14) A solid medicine or a solid food produced using a solid solution as set forth in any one of claims 1-13.

(15) A food containing the amino acid composition as set forth in claim 8.

(16) A method of manufacturing a solid solution composed of three amino acids of valine, isoleucine, leucine, which comprises concentrating an aqueous solution of 0.3-10.0 w/w % of valine, 0.3-6.5 w/w % of isoleucine, 0.3-4.0 w/w % of leucine at 40-90°C under a pressure of 3 kPa-30 kPa to crystallize, cooling the concentrated solution to 15-25°C, and then separating crystals.

(17) A manufacture method as set forth in claim 16, wherein, designating the total amount of isoleucine and leucine in the raw aqueous solution A[g], the weight of valine B[g], the weight ratio of valine in the produced solid solution V [w %], the effective distribution coefficient of valine against the total amount of isoleucine and leucine f[-], the crystallization yield of isoleucine and leucine in the produced solid solution Y [w %] .

The content of valine is controlled in the solid solution (composed of 5-85 w % of valine, 3-80 w % of isoleucine, 3-80 w % of leucine) based on the relationship;

$$V = 100fB / \{fB + A (100 - Y + fY) / 100\} \cdots 1$$

Wherein, designating the total amount of isoleucine and leucine in the raw aqueous solution A[g], the weight of valine B[g], the weight ratio of valine in the produced solid solution V [w %], the effective distribution coefficient of valine against the total amount of isoleucine and leucine f[-], the crystallization yield of isoleucine and leucine in the produced solid solution Y[w %].

(18) A manufacture method as set forth in claim 16, wherein, the effective distribution coefficient f[-] as set forth in claim 17 is calculated by the formula;

when the weight ratio of isoleucine to leucine is the produced solid solution is 2 or less,

$$0.226 \text{ x } [Val/(Ile + Leu)]_{\text{solid solution}} + 0.078 \cdots 2$$

when the weight ratio exceeds 2,

$$0.504 \text{ x } [Val/(Ile + Leu)]_{\text{solid solution}} + 0.029 \cdots 3$$

Herein, [Val/(Ile + Leu)] solid solution represents weight ratio [w %] of valine to the total amount of isoleucine and leucine in the solid solution.

(19) A method of producing a solid solution containing valine, isoleucine, leucine and at least one of acid selected from the group consisting of neutral aliphatic amino acid, neutral hydroxy amino acid, neutral acid amide amino acid, acidic amino acid, basic amino acid, hydroxy acid, which comprises crystallizing using a solution containing valine, isoleucine, leucine and at least one of acid selected from the group consisting of neutral aliphatic amino acid, neutral hydroxy amino acid, neutral acid amide amino acid, acidic amino acid, basic amino acid, hydroxy acid.

Effects of the Invention

In accordance with the present invention, a mixture of valine,isoleucine, leucine can be provided of which bitter taste is reduced and dissolution speed is improved. Upon using for foods, supplements, medicines, etc., addition of masking flavor, sweetening, acidulant, treatment with coating and the like can be obviated, or quantity to be used is saved greatly to facilitate the use of valine, isoleucine, leucine mixtures, and cost also can be reduced.

Furthermore, a mixture of valine, isoleucine, leucine can be provided of which the dissolution speed is remarkably improved, and thereby, handling in the processing step or of foods, supplements, medicines and the like can further be facilitated.

Brief Description of the Drawings

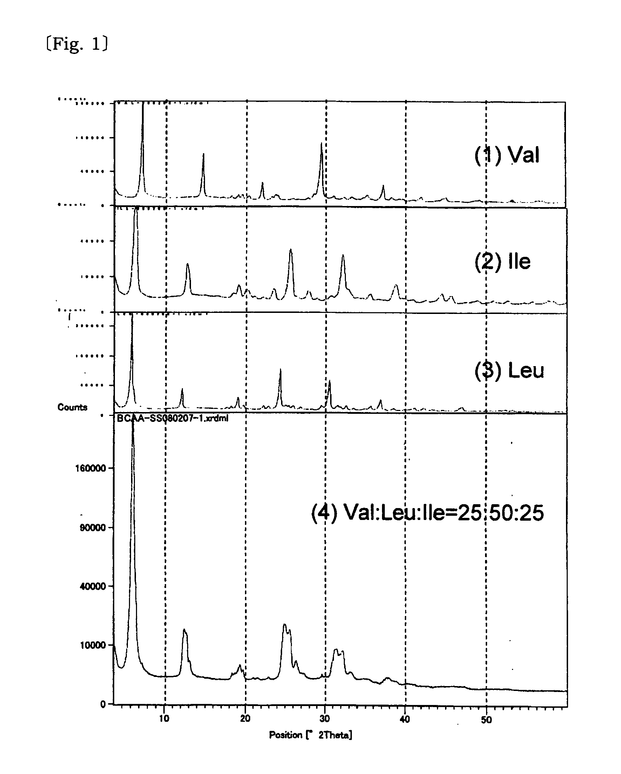

[FIG. 1] A powder X-ray diffraction pattern of a solid solution obtained in Preparation Example 1 of the invention and valine, isoleucine, leucine.

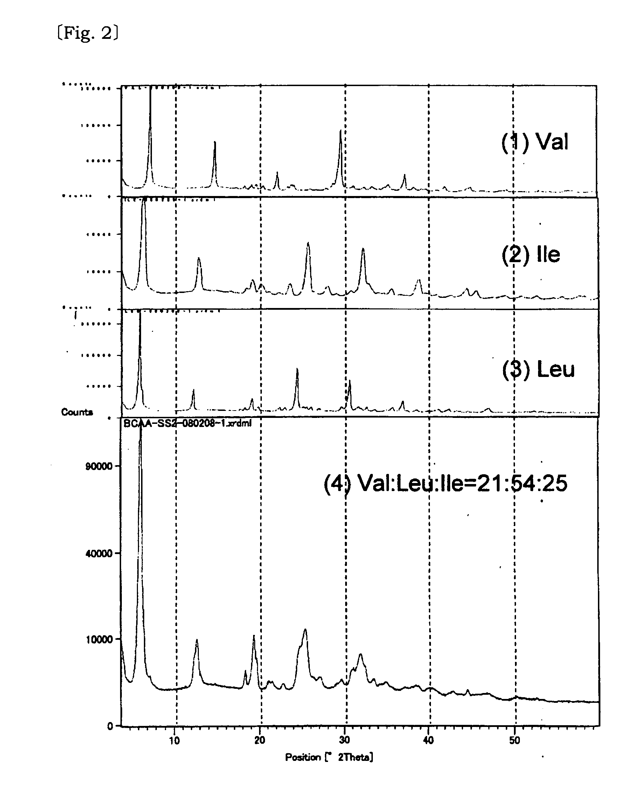

[FIG. 2] A powder X-ray diffraction pattern of a solid solution obtained in Preparation Example 2 of the invention and valine, isoleucine, leucine.

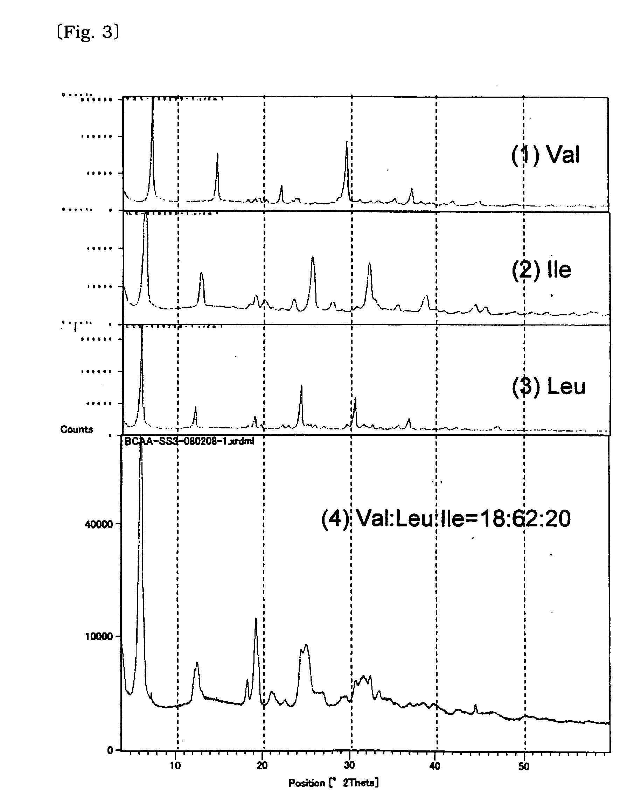

[FIG. 3] A powder X-ray diffraction pattern of a solid solution obtained in Preparation Example 3 of the invention and valine, isoleucine, leucine.

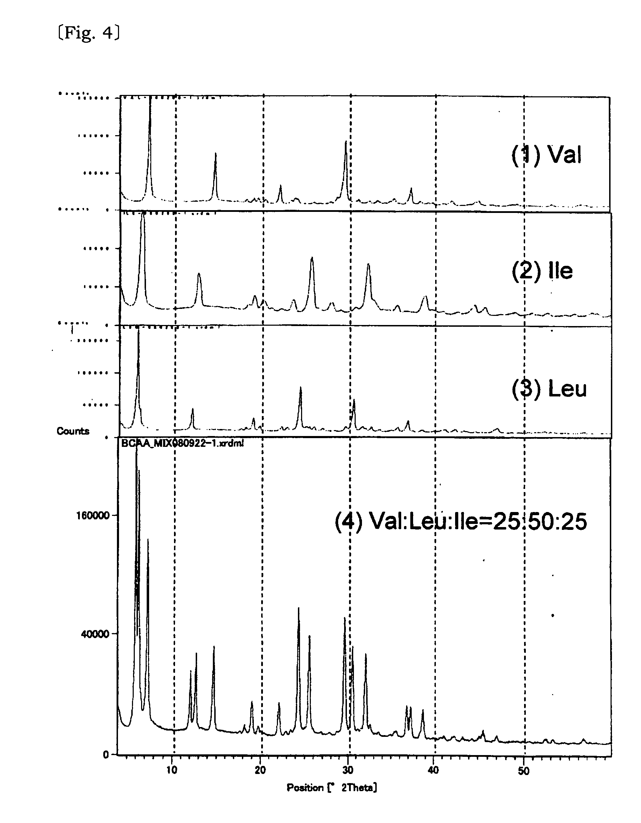

[FIG. 4] A powder X-ray diffraction pattern of a mixture obtained in Comparative Example 1 and valine, isoleucine, leucine.

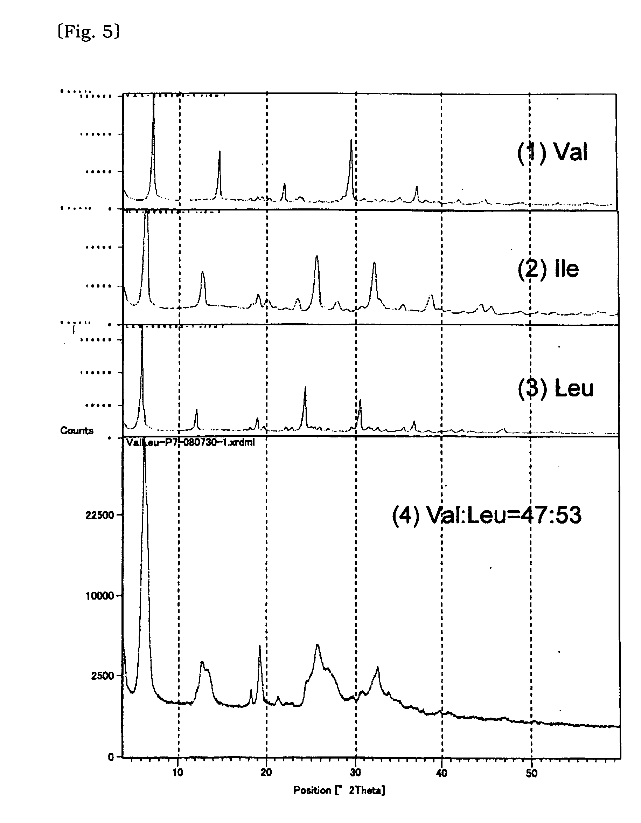

[FIG. 5] A powder X-ray diffraction pattern of a mixture obtained in Comparative Example 2 and valine, isoleucine, leucine.
[FIG. 6] A graph indicating dissolution speed of various samples.
[FIG. 7] A graph indicating the results of organoleptic evaluation of the solid solution against the mixture.

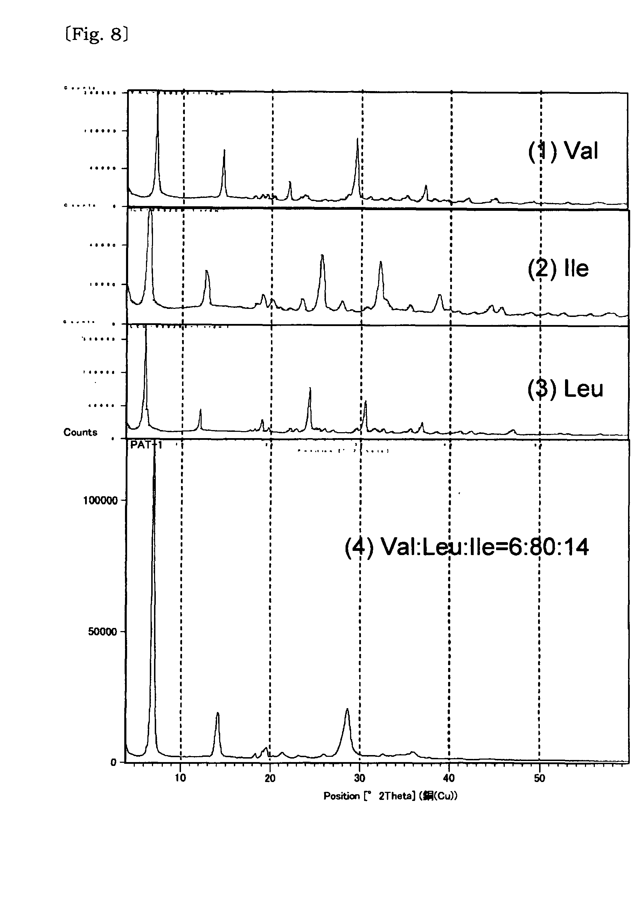

[FIG. 8] A powder X-ray diffraction pattern of a solid solution obtained in Preparation Example 5 of the invention and valine, isoleucine, leucine.

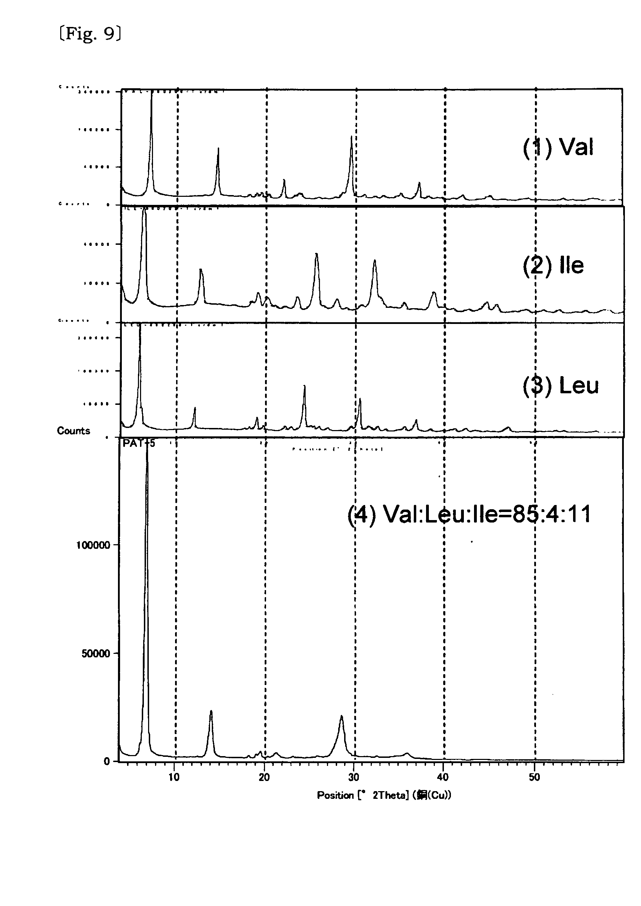

[FIG. 9] A powder X-ray diffraction pattern of a solid solution obtained in Preparation Example 6 of the invention and valine, isoleucine, leucine.

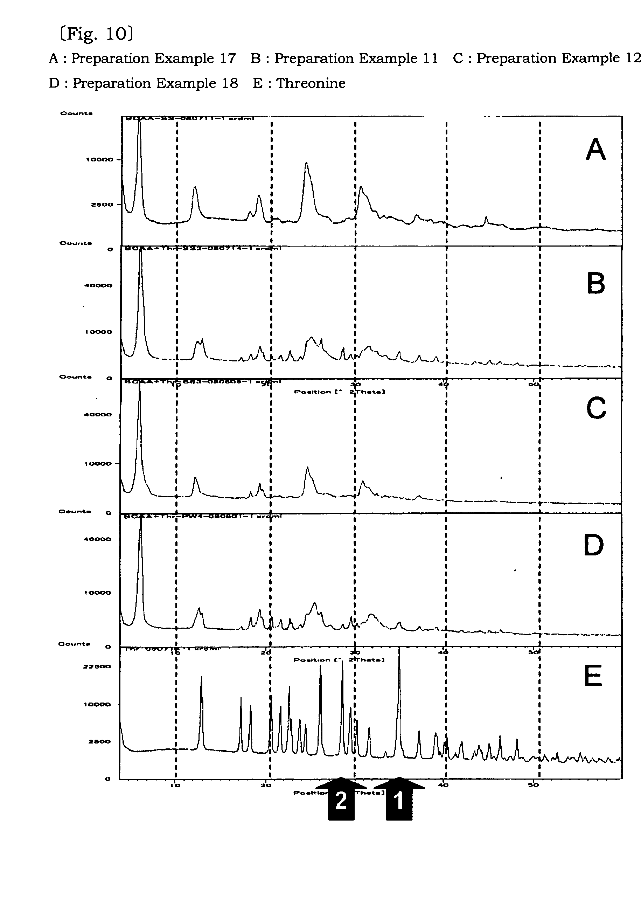

[FIG. 10] A powder X-ray diffraction pattern of solid solutions and powder mixtures obtained in Preparation Example 11, Preparation Example 12, Preparation Example 17, Preparation Example 18, threonine.

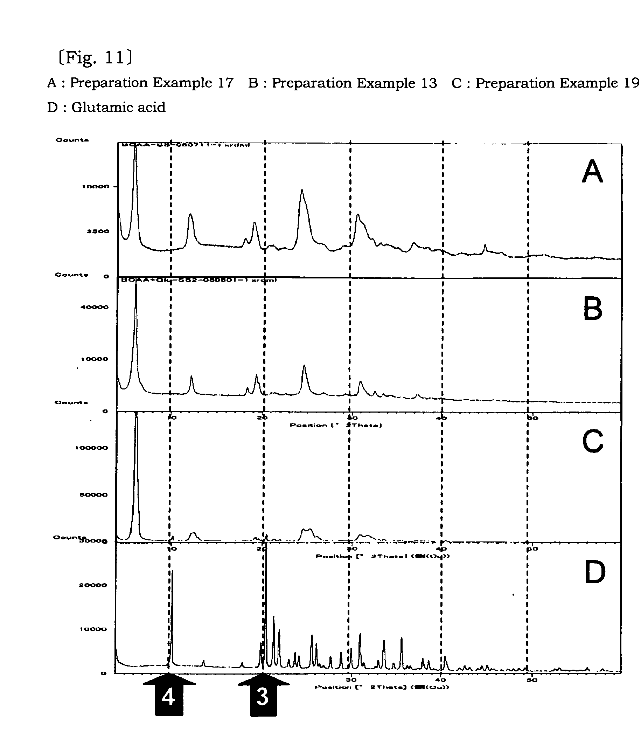

[FIG. 11] A powder X-ray diffraction pattern of solid solutions obtained in Preparation Example 13, Preparation Example 17 and glutamic acid.

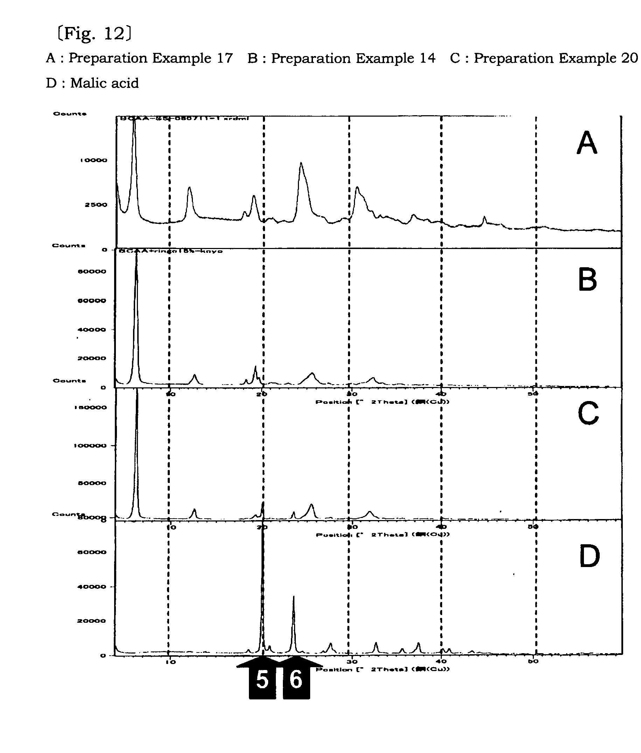

[FIG. 12] A powder X-ray diffraction pattern of solid solutions obtained in Preparation Example 14, Preparation Example 17 and malic acid.

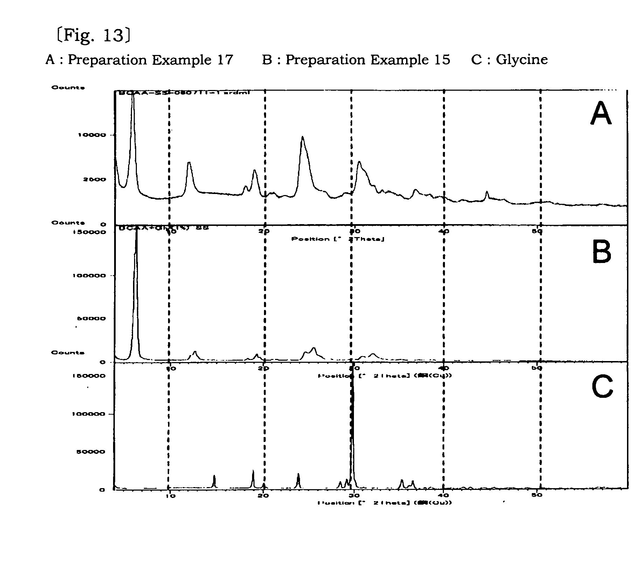

[FIG. 13] A powder X-ray diffraction pattern of solid solutions obtained in Preparation Example 15, Preparation Example 17 and glycine.

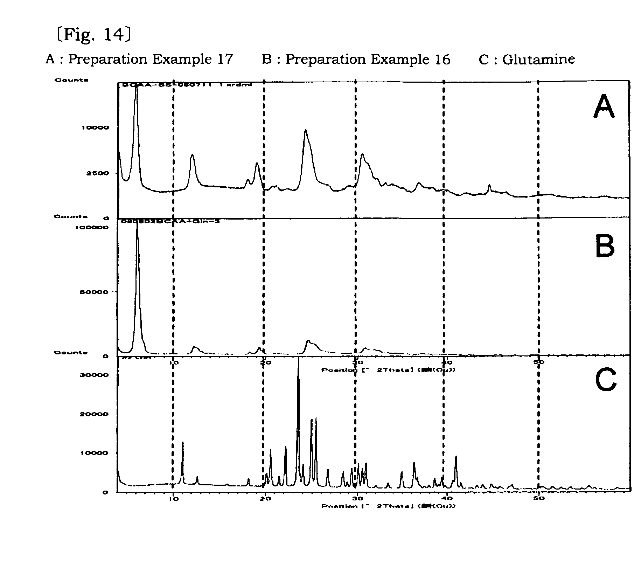

[FIG. 14] A powder X-ray diffraction pattern of solid solutions obtained in Preparation Example 16, Preparation Example 17 and glutamine.
[FIG. 15] A graph indicating dissolution speed of various samples at ordinary temperature.
[FIG. 16] A graph indicating dissolution speed of various samples at 50 degrees.

Mode for Carrying Out The Invention

Hereinafter, the present invention is explained in detail.
In the specification, valine, isoleucine, leucine are sometimes expressed BCAA (branched chain amino acids) as a general term. The neutral aliphatic amino acid in the invention means glycine, alanine, norleucine, homoleucine and the like. The neutral hydroxy amino acid in the invention means threonine, serine and the like. The neutral acid amide amino acid in the invention means asparagine monohydrate, glutamine and the like. The acidic amino acid in the invention means glutamic acid, aspartic acid and the like. The basic amino acid in the invention means arginine, lysine, histidine and the like. The hydroxy acid in the invention means malic acid, citric acid, tartaric acid and the like.
The mixture solution of dissolved valine, isoleucine, leucine amy be any one. For example, a solution prepared by dissolving crystals of valine, isoleucine, leucine into water, a mixture of solutions containing valine, isoleucine, leucine obtained from fermentation broths, a mixture of liquid(s) obtained from fermentation broth(s) and solution(s) prepared by dissolving crystals and the like are applicable.
The mixture solution of dissolved BCAA and at least one of acid selected from the group consisting of neutral aliphatic amino acid, neutral hydroxy amino acid, neutral acid amide amino acid, acidic amino acid, basic amino acid, hydroxy acid (hereinafter, these are referred to as the aforementioned amino acid and/or hydroxy acid) may also be any one. For example, a solution prepared by dissolving each amino acid or hydroxy acid, a mixture of solutions containing each amino acid or hydroxy acid obtained from fermentation broths, a mixture of liquid(s) obtained from fermentation broth(s) and solution(s) prepared by dissolving crystals and the like are applicable.
Each amino acid may be either L isomer, Disomer, DL racemic mixture, and however, L isomer is preferable in view of the applications to medicines or foods. Mixing ratio of valine, isoleucine, leucine is set according to the ratio of valine, isoleucine, leucine in the desired solid solution. When valine, isoleucine, leucine to be used are pure materials, the mixing ratio of respective amino acids is set the ratio in the desired solid solution, and the mixture solution is subjected to condensation crystallization to dryness. In the case of containing impurities to a certain degree, the crystallization yield of the solid solution is set so as to remove the impurities (this varies depending on raw materials, but can be set by a preliminary experimentation.), and it can be calculated from the volume and the concentration of valine, isoleucine, leucine of the mother liquor at the crystallization yield that the remainder forms the solid solution. For example, using 10g leucine containing 5 w/w % of impurities as a raw material, it is dissolved in water together with valine, leucine, and while conducting concentration crystallization, the operation is stopped at the crystallization of 50%, followed by solid-liquid separation. In this case, when 4.5g of leucine and 0.5g of impurities are found in the mother liquor, it results in the production of a solid solution not containing impurities. Like this, the removal of impurities and the preparation of the solid solution can be operated simultaneously.

The mixing ratio of the aforementioned amino acid and/or hydroxy acid with BCAA is set according to the ratio in the desired solid solution. In the case that the amino acid and the hydroxy acid to be used are pure materials, these are made at the ratio in the desired solid solution, and the mixture solution is subjected to condensation crystallization to dryness. In the case of containing impurities to a certain degree, the crystallization yield of the solid solution is set so as to remove the impurities (this varies depending on raw materials, but can be set by a preliminary experimentation.), and it can be calculated from the volume and the concentration of the amino acid and/or hydroxy acid of the mother liquor at the crystallization yield that the remainder forms the solid solution.

The crystallization method for producing crystals from a mixture solution of dissolved valine, isoleucine, leucine, or these and further dissolved the aforementioned amino acid and/or hydroxy acid, may be conventional to elevate the solution concentration to supersaturation to deposit crystals. For example, the applicable methods are the concentration crystallization method to remove solvent component by evaporation, the pH control method to vary pH from a high solubility pH region to a low solubility pH region to deposit crystals, the cooling crystallization method by cooling to transfer into a low solubility region to deposit crystals, the reaction crystallization method by adding a poor solvent, such as alcohol, to the solvent to lower solubility to deposit crystals, and the like.

The concentration crystallization, the cooling crystallization are preferable in the industrial viewpoint where the solvent and auxiliary material to be used specifically are only water, and particularly, in the case of the amino acids relataively difficult to dissolve in water, such as valine, leucine, isoleucine, it is desirable to combine the concentration crystallization and the cooling crystallization using a vacuum evaporator. Specifically, a method of producing a solid solution by the concentration crystallization of an aqueous solution of 0.3-10.0 w/w % (denominator is the weight of the aqueous solution, hereinafter the same.) of valine, 0.3-6.5 w/w % of isoleucine, 0.3-4.0 w/w % of leucine at 40°C-90°C under a pressure of 3 kPa-30 kPa, followed by cooling to ordinary temperature. The concentration of the aqueous solution upon charging is undesirable of less than 0.3 w/w % of valine, less than 0.3 w/w % of isoleucine, less than 0.3 w/w % of leucine is undesirable, because of not containing a necessary component. While, the concentration of the aqueous solution upon charging is undesirable of exceeding 10.0 w/w % of valine, 6.5 w/w % of isoleucine, 4.0 w/w % of leucine, 60.0 w/w % of glycine, 30.0 w/w % of alanine, 25.0 w/w % of threonine, 15.0 w/w % of glutamine, 17.0 w/w % of asparagine monohydrate, 6.5 w/w % of glutamic acid, 3.5 w/w % of aspartic acid, 120 w/w % of arginine, 150 w/w % of lysine, 18 w/w % of histidine, 40 w/w % of malic acid, 150 w/w % of citric acid, 140 w/w % of tartaric acid, because they cannot be dissolved. Moreover, the concentration of the aqueous solution upon charging is undesirable of 0.1 w/w % or less of at least one selected from the group consisting of neutral aliphatic amino acid, neutral hydroxy amino acid, neutral acid amide amino acid, acidic amino acid, basic amino acid, hydroxy acid, because the amount of the amino acid to be incorporated into crystals is small in the viewpoint of solubility.

Although the temperature and the degree of pressure reduction are controlled by the ability of evaporator, if the evaporator has a very high pressure reduction ability capable of concentrating at a pressure of 3 kPa, the concentration crystallization can be conducted at a solution temperature around 40°C. While, if the evaporator has an ability to heat up to 90°C, it is enough to evaporate even having a low pressure reduction ability. However, when production efficiency is taken into consideration, it is preferable to evaporate at a pressure of 30 kPa or lower.

It is possible to separate the crystals produced through the concentration crystallization, as they are. However, due to the lowering of the rate of recovery of valine, isoleucine,leucine (and further the aforementioned amino acid and/or hydroxy acid) caused by the high temperature, and/or in order to improve separation ability, the method of obtaining the solid solution after cooling up to 15-25°C.

The crystallization yield is set according to the purity of raw materials, the desired purity of the solid solution and the like, and it is possible to set 100%.

The produced material through the crystallization is a solid solution of valine, isoleucine, leucine, and when it contains the aforementioned amino acid and/or hydroxy acid, it is a solid solution composed of all of these materials. Since solid solution is in a state that two kinds or more of molecules are dissolved to each other to form a solid phase as a whole, when the peak(s) disappear from a spectrum of a solid obtained from single molecules measured by the powder X-ray diffraction method, the molecules are considered to be dissolved into between other molecules. Based on this consideration, valine, isoleucine, and further, the aforementioned amino acid and/or hydroxy acid are detected from the analytical values of constituents. In a powder X-ray diffraction spectrum, when a single peak of valine, isoleucine, leucine, or further. The aforementioned amino acid and/or hydroxy acid disappears, it is considered to be a solid solution. Solid solutions composed of valine, isoleucine, leucine are in a form of granules without corners, and characterized by that, in a powder X-ray diffraction pattern wherein the light source is CuKα rays, main peaks are shown at diffraction angles (2θ) of 6° -7° and 12° -14°. Herein, the main peaks mean the first peak, i.e. indicating (001) face of BCAA, and the second peak, i.e. indicating (002) face, from lower angle side.

The main peaks obtained from respective single molecules of the aforementioned amino acid and/or hydroxy acid are shown below. When the peaks appearing at these angles disappear, it is defined to be a solid solution not taking a single crystal structure. Moreover, when the peaks appearing at these angles are smaller compared with a simple powder mixture, it is considered to contain solid solution where inherent crystal structure has been broken.

Glycine diffraction angle (2θ) = 29.8°, 23.9°, alanine diffraction angle (2θ) = 20.6°, 28.9°, threonine diffraction angle (2θ) = 35.0°, 28.5°, glutamine diffraction angle (2θ) = 23.4° ,25.0°, asparagine monohydrate diffraction angle (2θ) = 29.2°, 18.1°, glutamic acid diffraction angle (2θ) = 10.3° ,20.5°, aspartic acid diffraction angle (2θ) = 23.8° ,11.9°, arginine diffraction angle (2θ) = 18.3°, 27.4°, citric acid diffraction angle (2θ) = 15.2°, 18.2°, malic acid diffraction angle (2θ) = 20.1°, 23.5°, tartaric acid diffraction angle (2θ) = 20.7°, 35.8°.

For the composition composed of BCAA and at least one type selected from the group consisting of the aforementioned amino acid and/or hydroxy acid, in the case that the main peaks of the aforementioned amino acid and/or hydroxy acid have a peak strength of 50% or less compared with a powder mixture with the same composition, preferably have a peak strength of 20% or less compared with a powder mixture with the same composition, more preferably have a peak strength of 5% or less, most preferably, no peak is detected, it is defined that the solid solution is contained.

The solid solution has a purity of 100% within the discriminatable range by a powder X-ray diffractometer commonly used using CuKα rays as the light source.

The solid solution of the invention is substantially composed of valine, isoleucine, leucine or further composed of the aforementioned amino acid and/or hydroxy acid, and such a solid solution occupies 97 w/w % or more of the total weight of solutes. Other solutes than the respective amino acids which can be listed are nonvaline, methionine and the like which are included during manufacture, but are not limited to them. The moisture content in the solid solution of the invention is usually 0.01-1.0 w/w % for dried matter, but the moisture content is not particularly limited so far as keeping the characteristics of the solid solution.

Furthermore, it is preferred that the solid solution of the invention has a ratio of 5-85 w/w % of valine, 3-80 w/w % of isoleucine, 3-80 w/w % of leucine. When the ratio of valine in the solid solution to be obtained is less than 5%, the solid solution is unfavorable, because the effect to improve bitter taste is insufficient. Whereas, the valine content of exceeding 85 w/w % is also unfavorable, because the improvement in dissolution speed is not found. Less than 3 w/w % of isoleucine, less than 3 w/w % of leucine, more than 80 w/w % of isoleucine, more than 80 w/w % of leucine are also unfavorable, because the improvement in dissolution speed is not found. In view of the improvement in taste, the improvement in dissolution speed, preferable ranges are 10-80 w/w % for valine, 5-75 w/w % for isoleucine, 5-75 w/w % for leucine, and more preferable ranges are 15-75 w/w % for valine, 10-60 w/w % for isoleucine, 10-70 w/w % for leucine.

Further, in the case of containing the aforementioned amino acid and/or hydroxy acid, it is preferred that the solid solution of the invention has a ratio of 5-85 w/w % of valine, 3-80 w/w % of isoleucine, 3-80 w/w % of leucine, 0.01-50 w/w % of the aforementioned amino acid and/or hydroxy acid. Less than 0.01 w/w % of the aforementioned amino acid and/or hydroxy acid is unfavorable because the improvement in dissolution speed is not found. While, for the purpose of improving solubility of BCAA, to contain more than 50 w/w % of the other amino acids is unfavorable.

In view of producing the solid solution, when containing the aforementioned amino acid and/or hydroxy acid, it is preferred to contain 0.1-10 w/w % of neutral aliphatic amino acid, 0.01-30 w/w % of neutral hydroxy acid, 0.01-25 w/w % of neutral acid amide amino acid, 0.01-45 w/w % of acidic amino acid. It is further preferred to contain in a concentration range of 0.1-2 w/w % of neutral aliphatic amino acid, 0.5-5 w/w % of neutral hydroxy amino acid, 0.5-5 w/w % of neutral acid amide amino acid, 0.1-10 w/w % of acidic amino acid, 0.1-10 w/w % of basic amino acid, 0.1-15 w/ w % of hydroxy acid.

The mechanism of forming the solid solution, even further adding the aforementioned amino acid and/or hydroxy acid to the three type of valine, isoleucine, leucine, is presumed as follows: The formation of solid solution is in a state that two types or more molecules form a regular crystal structure one another, and at that time, small molecules (polarity is weak, and constituent atoms are present at a short distance) are tend to enter between large molecules (having strong polarity and spreading). Therefore, it is considered that, as to alanine, glycine having a relatively small molecular size, the amount of amino acids capable of forming solid solution is small.

Upon preparing a solid solution composed of BCAA and at least one type selected from the group consisting of the aforementioned amino acid and/or hydroxy acid, the incorporation into crystals is theorized as follows: Amino acids having low solubility are much in the mother liquor, while crystallization yield is small in the crystallization process, and the amount incorporated into the crystals is small compared with the ratio in the charging quantities. However, when crystallization yield increases, the concentration of the amino acids having low solubility is raised in the mother liquor, and the incorporation into the crystals increases. As a result, the higher the solubility in water of an amino acid is, the more the amino acid located toward the outside of crystals.

The solid solution composed of valine, isoleucine, leucine can be manufactured with a desired composition by the following method.

The following matter was found as to the solid solution produced by the crystallization from an aqueous solution containing valine, isoleucine, leucine. The weight ratio of isoleucine to leucine in the solid solution is proportional to the weight ratio of isoleucine to leucine in the aqueous raw material solution, regardless of the valine content (5-85%) in the same solid solution. Namely, [Ile/Leu] solid solution = 0.87-1.00 vs [Ile/Leu] raw material Valine content [w %] in the solid solution can be controlled by using an effective distribution coefficient of valine against the total amount of isoleucine and leucine in the same solid solution.

That is, it is possible that the weight ratio of isoleucine to leucine in the solid solution can be determined by the weight ratio of isoleucine to leucine in the raw material, and that the valine content in the solid solution can be adjusted by using the effective distribution coefficient of valine against the total amount of isoleucine and leucine.

As to the effective distribution coefficient of valine against the total amount of isoleucine and leucine and the valine content in the solid solution in the above paragraph, details are described below.

In the raw material, when the total amount of isoleucine and leucine is A [g], the amount of valine is B [g], the effective distribution coefficient of valine against the total amount of isoleucine and leucine is f [-], the crystallization yield of the total amount of isoleucine and leucine is Y [%], and the amount of valine contained in the solid solution after crystallization is b [g], from the definition of effective distribution coefficient;

$$f = \{b/(AY/100)\}/\{(B\text{-}b)/(A\text{-}AY/100)\}$$

When this formula is arranged as to b;

$$b = fYB/(100\text{-}Y+fY) \quad \cdots \quad 4$$

When the Val content (%) in the solid solution is V, since V = b/(b+AY/100) x 100, the formula 4 is substituted, and rearranged to obtain the formula 1.

$$V\,(\%) = 100fB/\{fB+A(100\text{-}Y+fY)/100\} \quad \cdots \quad 1$$

Accordingly, the Val content (%) in the solid solution is determined by the respective amount of isoleucine, leucine, valine in the raw material, and the effective distribution coefficient, and the crystallization of the total amount of isoleucine and leucine. When the formula 1 is transformed as to Y;

$$Y = \{V(A+fB)\text{-}fB \text{ x } 100\} \text{ x } 100/\{AV(1\text{-}f)\} \quad \cdots \quad 5$$

Accordingly, for the desired Val content (%) of the solid solution, the necessary crystallization yield of the total amount of isoleucine and leucine can be determined by the calculation using the formula 5. Alternatively, it is also possible for the desired Val content (%), the crystallization yield is fixed to determine the necessary amount of valine in the raw material. This is explained concretely in the examples ([Preparation Example 7] - [Preparation Example 10]).

It is known that valine exhibits also sweetness in addition to bitter taste, compared with isoleucine, leucine, and that the solubility is higher, compared with isoleucine, leucine. From this, the mechanism of reducing bitter taste by producing solid solution is presumed as follows: Among the three branched chain amino acids, valine having a high solubility in water is present much in the mother liquor, while the crystallization yield is low, and the incorporated amount into crystals is small, compared with the ratio in the charging quantities. However, when the crystallization becomes high, the valine concentration in the mother liquor is raised to increase the incorporation into crystals. Therefore, in the order of higher solubility in water, i.e. valine > isoleucine > leucine, the more the amino acid exists toward the outside of crystals. While, valine is positioned as an amino acid exhibiting sweetness compared with isoleucine, leucine, and its stimulus threshold is 150mg/dl which is higher compared with 90mg/dl of isoleucine. Accordingly, when dissolved in the mouth, its taste is not felt so sensitive than isoleucine. It is presumed that, in the solid solution where valine exists much on the outside, the bitter taste is reduced compared with a conventional powder mixture of valine, isoleucine, leucine.

To be rapid the dissolution speed of the solid solution of the invention compared with powder mixture is presumed as follows: It is considered that, in the solid solution where valine, isoleucine, leucine molecules are mixed together, intermolecular forces are weak, and free energy of mixing solid and water when dissolving in water is small. And, further considered that the free energy of mixing of the solid solution of three amino acids is smaller than the solid solution of two amino acids.

From the above presumption, when the ratio of valine in the solid solution to be produced is less than 5 w/w %, the effect of improving bitter taste is insufficient, while when exceeding 85 w/w %, the dissolution speed is not improved, and therefore, being unfavorable. Moreover, in view of dissolution speed, it is unfavorable that the ratio of isoleucine or leucine exceeds 80 w/w % as alone of which the dissolution speed is slower than valine.

To be rapid the dissolution of the solid solution of the invention compared with powder mixture, the solid solution of three types, valine, leucine, isoleucine is presumed as follows: It is considered that, in the solid solution where foreign molecules are mixed together in addition to valine, isoleucine, leucine, intermolecular forces are further weak, and free energy of mixing solid and water when dissolving in water is small. And, further considered that the free energy of mixing of the solid solution of four amino acids is smaller than the solid solution of three amino acids.

From the object of the invention, less than 50 w/w % of BCAA contained in the solid solution is unfavorable, because the significance of improving dissolution speed is not achieved. Moreover, in view of dissolution speed, it is unfavorable that, when the ratio of one amino acid of BCAA (valine, isoleucine, leucine) exceeds 80 w/w % as alone, mixing in the crystal structure becomes insufficient.

The size of the solid solution of the invention is preferably 50-1000$\mu$m as a mean particle size. The mean particle size as used herein refers to the number median particle size of sieve diameter D50. In view of dissolution speed taking melting ability in the mouth of solid foods and usability as solid beverages into consideration, too large crystals are unsuitable for practical use. Too small crystals are also unsuitable, due to being choked by dusting upon eating and dusting loss upon manufacturing.

As the solid medicines using the solid solution of the invention, oral medicines for irrhotics produced by granulating the solid solution of the invention together with sweetener and/or binder, powdered medicines produced by mixing the solid solution with sweetener and/or excipient, and the like can be listed. As the solid foods using the solid solution of the invention, powder-shaped or granular amino acid supplements produced by mixing the solid solution together with sweetener, acidulant, excipient and/or the other components, and granulated thereof.

Examples

The present invention is further explained in accordance with Examples and Comparative Examples.

[Preparation Example 1]

10g of L-valine, 10g of L-isoleucine, 20g of L-leucine were dissolved in 1000ml of water at 80°C, and the solution was concentrated by a vacuum evaporator (manufactured by Tokyo Rikagaku Kikai Co., Ltd.) at 70°C at 127 kPa for about 3 hours to evaporate water completely to collect granular residue thus produced. X-ray diffraction spectrum of the residue were measured to confirm that this is a solid solution.

[Preparation Example 2]

10g of L-valine, 10g of L-isoleucine, 20g of L-leucine were dissolved in 1000ml of water at 80°C, and the solution was concentrated by a vacuum evaporator at 70°C at 8 kPa (hPa?) for about 1.5 hours after evaporating 810ml of water, the concentrated solution was cooled to ordinary temperature for about 0.5 hour, and solid was separated from the solution. The solid was air-dried to collect granular solid solution.

[Preparation Example 3]

8g of L-valine, 8g of L-isoleucine, 24g of L-leucine were dissolved in 1200ml of water at 80°C, and the solution was concentrated by a vacuum evaporator at 70°C at 60mmHg for about 2 hours. After evaporating 1050ml of water, the concentrated solution was cooled to ordinary temperature for about 0.5 hour, and solid was separated from the solution. The solid was air-dried to collect granular solid solution.

[Preparation Example 4]

5g of L-valine, 5g of L-isoleucine, 10g of L-leucine were dissolved in 500ml of water at 80°C, and 500ml of ethanol at 25 degrees was added to the solution, followed by cooling to ordinary temperature for about 0.5 hour. Solid was separated from the mixed solution, and the solid was air-dried to collect powder-shaped solid solution.

[Comparative Example 1]

5g of L-valine, 5g of L-isoleucine, 10g of L-leucine were pulverized and mixed by a blender mill.

[Comparative Example 2]

10g of L-valine, 10g of L-isoleucine were dissolved in 450ml of water at 80°C, and the solution was concentrated by a vacuum evaporator at 70°C at 60 kPa for about 1 hour. After evaporating 350ml of water, the concentrated solution was cooled to ordinary temperature, and solid was separated from the solution. The solid was air-dried to collect solid solution of two amino acids.

Amino acid composition, powder X-ray diffraction spectrum, dissolution speed and mean particle size of the solid solutions of Preparation Example 1-3, Comparative Example 1, Comparative Example 2, pulverized L-valine, pulverized L-isoleucine, pulverized L-leucine are shown in Table 1.

The amino acid composition was analyzed by an amino acid analyzer ("L-8800", manufactured by Hitachi Ltd.). The powder x-ray diffraction spectrum was measured by a powder x-ray diffraction apparatus ("X' Pert-Pro-MPD; PAN-alytical B.V.") using Cub$\alpha$ rays (40 kV, 30 mA) at $2\theta$ position of 4° - 60°.

As to the dissolution speed, 1g of powder was dropped into 100ml of warm water at 50°C with stirring at a number of revolutions of 550 rpm (using a 200ml beaker and a magnetic stirrer), and the time until dissolving completely was measured. The time was employed as an indicator of the dissolution speed.

As to the mean particle size, D50 was measured by a digital image analysis type particle size distribution meter (Ana Tec As Norway).

[Table 1]

| Sample | Amino Acid Composition (ratio by weight) | X-ray Diffraction / Spectrum | Dissolution Speed | Mean Particle Size |
|---|---|---|---|---|
| Prep. Ex. 1 | Val:Leu:Ile=25:50:25 | (4) in Fig. 1 | 55 sec. | 600 $\mu$m |
| Prep. Ex. 2 | Val:Leu:Ile=21:54:25 | (4) in Fig. 2 | 34 sec. | 350 $\mu$m |
| Prep. Ex. 3 | Val:Leu:Ile=18:62:20 | (4) in Fig. 3 | 52 sec. | 220 $\mu$m |
| Comp. Ex. 1 | Val:Leu:Ile=25:50:25 | (4) in Fig. 4 | 110 sec. | 310 $\mu$m |
| Comp. Ex. 2 | Val:Leu=47:53 | (4) in Fig. 5 | 98 sec. | 250 $\mu$m |
| Val | Val = 100 w/w % | (1) in Fig. 1,2,3,4 and 5 | 118 sec. | 210 $\mu$m |
| Ile | Ile= 100 w/w % | (2) in Fig. 1,2,3,4 and 5 | 129 sec. | 400 $\mu$m |
| Leu | Leu= 100 w/w % | (3) in Fig. 1,2,3,4 and 5 | 130 sec. | 280 $\mu$m |

Dissolution speed

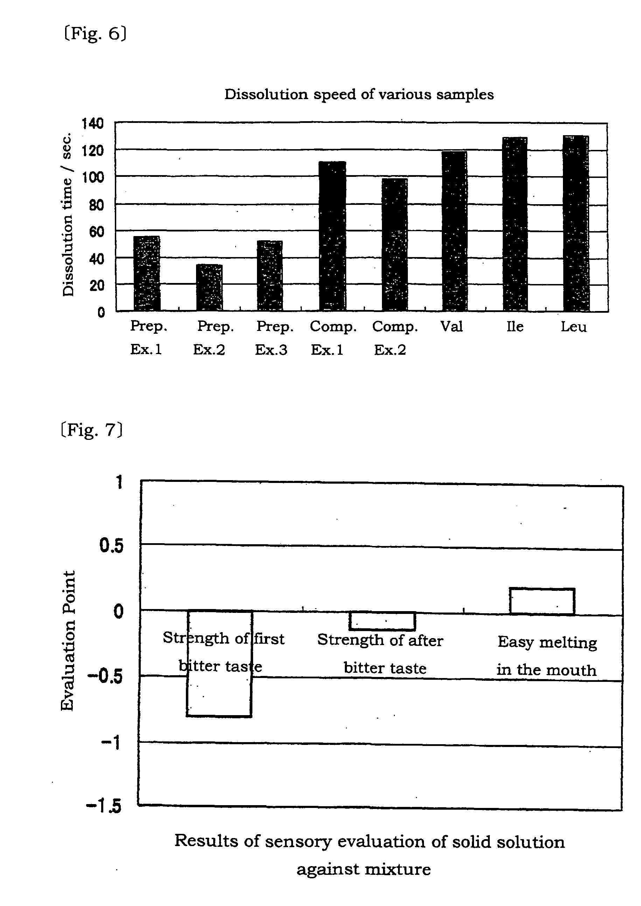

A dissolution speed of each sample is shown in Figure 6. From the figure, it was found that, even considering the dispersion of the mean particle sizes of various samples, the dissolution speed of the solid solutions is still remarkably fast.

Sensory Evaluation

Sensory evaluation was carried out as to the solid solution of Preparation Example 2. The simple mixture in Comparative Example 1 was used as a criterion, the taste of the solid solutions was divided into "strength of first bitter taste", "strength of after bitter taste", "easy melting in the mouth", and were evaluated organoleptically by 5 special panel members. The evaluation was conducted designating "very strong" as +3, "equivalent" as 0, "very weak" as -3, and represented by numerical values at 0.1 intervals. The results are shown in Figure 7.

As to the evaluation item "strength of first bitter taste", it was confirmed that the solid solution (Preparation Example 2) is weaken than the powder mixture (Comparative Example 1) with a significant difference of the risk factor being 5 w/w % or less. As to the evaluation item "easy melting in the mouth", it was indicated that the solid solution is easier with a significant difference of the risk factor being 10 w/w % or less.

[Preparation Example 5]

1.2g of L-valine, 2.8g of L-isoleucine, 16g of L-leucine were dissolved in 650ml of water at 80 °C , and the solution was concentrated by a vacuum evaporator (manufactured by Tokyo Rikagaku Kikai Co., Ltd.) at 70°C at 127 kPa for about 3 hours to evaporate water completely to collect granular residue thus produced. X-ray diffraction spectra of the residue were measured to confirm that this is a solid solution.

[Preparation Example 6]

17g of L-valine, 0.8g of L-isoleucine, 2.2g of L-leucine were dissolved in 350ml of water at 80 °C , and the solution was concentrated by a vacuum evaporator (manufactured by Tokyo Rikagaku Kikai Co., Ltd.) at 70°C at 127 kPa for about 2 hours to evaporate water completely to collect granular residue thus produced. X-ray diffraction spectra of the residue were measured to confirm that this is a solid solution.

[Comparative Example 3]

0.6g of L-valine, 1.4g of L-isoleucine, 8g of L-leucine, which had been pulverized by a blender mill, were mixed.

[Comparative Example 4]

8.5g of L-valine, 1.1g of L-isoleucine, 0.4g of L-leucine, which had been pulverized by a blender mill, were mixed.
Dissolution speed and mean particle size of the solid solutions of Preparation Examples 5, 6 and the powder of Comparative Examples 3, 4 and shown in Table 2.

[Table 2]

| Sample | Amino Acid Composition (ratio by weight) | X-ray Diffraction / Spectrum | Dissolution Speed | Mean Particle Size |
|---|---|---|---|---|
| Prep. Ex. 5 | Val:Leu:Ile=6:80:14 | (4) in Fig. 8 | 95 sec. | 390 μm |
| Prep. Ex. 6 | Val:Leu:Ile=85:4:11 | (4) in Fig. 9 | 59 sec. | 340 μm |
| Comp. Ex. 3 | Val:Leu:Ile=6:80:14 | - | 158 sec. | 290 μm |
| Comp. Ex. 4 | Val:Leu:Ile=85:4:11 | - | 69 sec. | 290 μm |

[Preparation Example 7]

Target composition ratio of the solid solution: valine/isoleucine/leucine = 27/32/40 (w %).
23.1g of L-valine, 17.5g of L-isoleucine, 21.0g of L-leucine were dissolved in 1200ml of water at 80°C. In the solution, the total amount of isoleucine and leucine was 38.5g, and isoleucine/leucine was 0.83. While, since the ratio by weight of valine to the total amount of isoleucine and leucine in the target solid solution was 0.38, and isoleucine/ leucine < 2, the effective distribution coefficient of valine against the total amount of isoleucine and leucine was calculated to be 0.163 according to the formula 2. These values and 27 (w %) of the target valine content were substituted in the calculation formula 5 to determine the crystallization yield of the total amount of isoleucine and leucine as 88%. Thereupon, so as to obtain the solid solution at the same crystallization yield, the above solution was concentrated by a vacuum evaporator to evaporate moisture, followed by cooling to room temperature, and solid was separated from the solution by a centrifugal filter to collect the solid solution. The crystallization yield of isoleucine and leucine in total was 88.3%.
The composition ratio (w %) of the obtained solid solution was valine/isoleucine/leucine = 27.4/31.9/40.7 and was consistent well with the target value. Moreover, isoleucine/leucine was 0.78 which corresponds to 94% of the ratio in the raw material solution and was consistent well with the calculated value described in (1).

[Preparation Example 8]

Target composition ratio of the solid solution: valine/isoleucine/leucine = 27/32/40 (w %).

137.3g of L-valine, 90.0g of L-isoleucine, 105.5g of L-leucine were dissolved in 7000ml of water at 80°C. In the solution, the total amount of isoleucine and leucine was 195.5g, and isoleucine/leucine was 0.85. While, since the ratio by weight of valine to the total amount of isoleucine and leucine in the target solid solution was 0.38, and isoleucine/leucine < 2, the effective distribution coefficient of valine against the total amount of isoleucine and leucine was calculated to be 0.163 according to the formula 2. These values and 27 (w %) of the target valine content were substituted in the calculation formula 5 to determine the crystallization yield of the total amount of isoleucine and leucine as 83%. Thereupon, so as to obtain the solid solution at the same crystallization yield, the above solution was concentrated by a vacuum evaporator to evaporate moisture, followed by cooling to room temperature, and solid was separated from the solution by a centrifugal filter to collect the solid solution. The crystallization yield of isoleucine and leucine in total was 82.9%.

The composition ratio (w %) of the obtained solid solution was valine/isoleucine/leucine = 27.2/31.9/40.9 and was consistent well with the target value. Moreover, isoleucine/leucine was 0.78 which corresponds to 92% of the ratio in the raw material solution and was consistent well with the calculated value.

[Preparation Example 9]

Target composition ratio of the solid solution: valine/isoleucine/leucine = 25/25/50 (w %).
20.0g of L-valine, 15.0g of L-isoleucine, 30.0g of L-leucine were dissolved in 1600ml of water at 80°C. In the solution, the total amount of isoleucine and leucine was 45.0g, and isoleucine/leucine was 0.5. While, since the ratio by weight of valine to the total amount of isoleucine and leucine in the target solid solution was 0.33, and isoleucine/leucine < 2, the effective distribution coefficient of valine against the total amount of isoleucine and leucine was calculated to be 0.153 according to the formula 2. These values and 25 (w %) of the target valine content were substituted in the calculation formula 5 to determine the crystallization yield of the total amount of isoleucine and leucine as 94%. Thereupon, so as to obtain the solid solution at the same crystallization yield, the above solution was concentrated by a vacuum evaporator to evaporate moisture, followed by cooling to room temperature, and solid was separated from the solution by a centrifugal filter to collect the solid solution. The crystallization yield of isoleucine and leucine in total was 94.7%.

The composition ratio (w %) of the obtained solid solution was valine/isoleucine/leucine = 25.1/24.8/50.1 and was consistent well with the target value. Moreover, isoleucine/leucine was 0.50 which corresponds to 99% of the ratio in the raw material solution and was consistent well with the calculated value.

[Preparation Example 10]

Target composition ratio of the solid solution: valine/isoleucine/leucine = 31/54/15 (w %).
13.5g of L-valine, 15.4g of L-isoleucine, 3.9g of L-leucine were dissolved in 700ml of water at 80°C. In the solution, the total amount of isoleucine and leucine was 19.3g, and isoleucine/leucine was 3.9. While, since the ratio by weight of valine to the total amount of isoleucine and leucine in the target solid solution was 0.45, and isoleucine/leucine > 2, the effective distribution coefficient of valine against the total amount of isoleucine and leucine was calculated to be 0.26 according to the formula 3. These values and 31 (w %) of the target valine content were substituted in the calculation formula 5 to determine the crystallization yield of the total amount of isoleucine and leucine as 81%. Thereupon, so as to obtain the solid solution at the same crystallization yield, the above solution was concentrated by a vacuum evaporator to evaporate moisture, followed by cooling to room temperature, and solid was separated from the solution by a centrifugal filter to collect the solid solution. The crystallization yield of isoleucine and leucine in total was 81.6%.

The composition ratio (w %) of the obtained solid solution was valine/isoleucine/leucine = 31.4/53.6/14.9 and was consistent well with the target value. Moreover, isoleucine/leucine was 3.6 which corresponds to 92% of the ratio in the raw material solution and was consistent well with the calculated value.

[Preparation Example 11]

5g of L-valine, 5g of L-isoleucine, 10g of L-leucine, 5g of L-threonine were dissolved in 500ml of water at 80°C, and the solution was concentrated by a vacuum evaporator at 70°C at 127 kPa for about 1.5 hours to evaporate 459ml of water. Thereafter, the concentrated solution was cooled to ordinary temperature for about 0.5 hour, solid was separated from the solution. The solid was air-dried to collect 23.4g of granular solid solution.

[Preparation Example 12]

5g of L-valine, 5g of L-isoleucine, 10g of L-leucine, 2g of L-threonine were dissolved in 500ml of water at 80°C, and the solution was concentrated by a vacuum evaporator (manufactured by Tokyo Rikagaku Kikai Co., Ltd.) at 70°C at 127 kPa for about 1.5 hours to evaporate 402ml of water. Thereafter, the concentrated solution was cooled to ordinary temperature for about 0.5 hour, solid was separated from the solution. The solid was air-dried to collect 17.1 g of granular solid solution.

[Preparation Example 13]

5g of L-valine, 5g of L-isoleucine, 10g of L-leucine, 2.5g of glutamic acid were dissolved in 500ml of water at 80°C, and the solution was concentrated by a vacuum evaporator at 70°C at 127 kPa for about 1.5 hours to evaporate 411ml of water. Thereafter, the concentrated solution was cooled to ordinary temperature for about 0.5 hour, solid was separated from the solution. The solid was air-dried to collect 18g of granular solid solution.

[Preparation Example 14]

8g of L-valine, 9.6g of L-isoleucine, 12g of L-leucine, 5g of malic acid were dissolved in 500ml of water at 80°C, and the solution was concentrated by a vacuum evaporator at 70°C at 127 kPa for about 2 hours to evaporate water completely to collect granular residue thus produced.

[Preparation Example 15]

5g of L-valine, 5g of L-isoleucine, 10g of L-leucine, 0.2g of L-glycine were dissolved in 500ml of water at 80°C, and the solution was concentrated by a vacuum evaporator at 70 degrees at 127mmHg for about 2 hours to evaporate water completely to collect granular residue thus produced.

[Preparation Example 16]

2.5g of L-valine, 2.5g of L-isoleucine, 5g of L-leucine, 0.25g of glutamine were dissolved in 300ml of water at 80°C, and the solution was concentrated by a vacuum evaporator at 70°C at 127 kPa for about 2 hours to evaporate water completely to collect granular residue thus produced.

[Preparation Example 17]

Similar to Preparation Examples 1 to 3, L-valine, L-isoleucine, L-leucine were dissolved in warm water, and the solution was concentrated to crystallize by a vacuum evaporator. Solid was separated from the concentrated solution, and the solid was dried. A solid solution having a weight ratio of valine:leucine:isoleucine being 12:62:25 was obtained.

[Preparation Example 18]

Similar to Preparation Examples 1 to 3, L-valine, L-isoleucine, L-leucine were dissolved in warm water, and the solution was concentrated to crystallize by a vacuum evaporator. Solid was separated from the concentrated solution, and the solid was dried. A solid solution having a weight ratio of valine:leucine:isoleucine being 16:60:24 was obtained. To 95 w/w % of the solid solution, L-threonine was mixed at a rate of 5 w/w %.

[Preparation Example 19]

Similar to Preparation Examples 1 to 3, L-valine, L-isoleucine, L-leucine were dissolved in warm water, and the solution was concentrated to crystallize by a vacuum evaporator. Solid was separated from the concentrated solution, and the solid was dried. A solid solution having a weight ratio of valine:leucine:isoleucine being 20:55:25 was obtained. To 83 w/w % of the solid solution, L-glutamic acid was mixed at a rate of 17 w/w %.

[Preparation Example 20]

20g of L-valine, 15g of L-isoleucine, 30g of L-leucine were dissolved in 1600ml of water at 80 °C, and the solution was concentrated by a vacuum evaporator at 70°C at 127 kPa, until crystals were produced at a rate of 94.7%. Thereafter, the concentrate was cooled to ordinary temperature, and solid was separated. The solid was dried to collect 42.6g of a granular solid solution. To the BCAA solid solution, malic acid was mixed at a rate of 20 w/w %.

Composition ratio, powder X-ray diffraction spectrum, dissolution speed and mean particle size of the solid solutions composed of four types of materials of Preparation Examples 11-16, the compositions composed of three types of materials of valine, leucine, isoleucine of the solid solutions to which a fourth material was added of Preparation Examples 17-20, pulverized L-threonine, pulverized L-glutamic acid, pulverized L-malic acid, pulverized glycine, pulverized L-glutamine are shown in Table 3.

The amino acid composition was analyzed by an amino acid analyzer ("L-8800", manufactured by Hitachi Ltd.). The powder X-ray diffraction spectrum was measured by a powder X-ray diffraction apparatus ("X' Pert-Pro-MPD; PANalytical B.V.") using CuKα rays (40 Kv, 30 mA) at 2 θ position of 4° -60°.

As to the dissolution speed, 1g of powder was dropped into 100ml of warm water at 50°C or water at ordinary temperature with stirring at a number of revolutions of 550 rpm (using a 200ml beaker and a magnetic stirrer), and the time until dissolving completely was measured. A mean value of twice measurement was employed as an indicator of the dissolution speed.

As to the mean particle size, D50 was measured by a digital image analysis type particle size distribution meter (Ana Tec As Norway).

[Table 3]

| Sample | Amino Acid Composition (ratio by weight) | X-ray Diffraction / Spectrum |
|---|---|---|
| Prep. Ex. 11 | Val:Leu:Ile:Thr=15:51:23:11 | B in Fig. 10 |
| Prep. Ex. 12 | Val:Leu:Ile:Thr=15:56:23:5 | C in Fig. 10 |
| Prep. Ex. 13 | Val:Leu:Ile:Glu=17:46:20:17 | B in Fig. 11 |
| Prep. Ex. 14 | Val:Leu:Ile:malic acid=20:40:20:20 | B in Fig. 12 |
| Prep. Ex. 15 | Val:Leu:Ile:Gly=23:51:26:0.4 | B in Fig. 13 |
| Prep. Ex. 16 | Val:Leu:Ile:Gln=24:50:25:0.3 | B in Fig. 14 |
| Prep. Ex. 17 | Val:Leu:Ile=12:62:25 | A in Fig. 10-14 |
| Prep. Ex. 18 | Val:Leu:Ile:Thr=15:56:23:5 | D in Fig. 10 |
| Prep. Ex. 19 | Val:Leu:Ile:Glu=17:46:20:17 | C in Fig. 11 |
| Prep. Ex. 20 | Val:Leu:Ile:malic acid=20:40:20:20 | C in Fig. 12 |
| Threonine | Thr =100 w/w % | E in Fig. 10 |
| Grutamic Acid | Glu =100 w/w % | D in Fig. 11 |
| Marlic Acid | Malic acid =100 w/ w % | D in Fig. 12 |
| Glycine | Gly = 100 w/w % | D in Fig. 13 |
| Glutamine | Gln = 100 w/w % | D in Fig. 14 |

[Preparation Example 21]

Similar to Preparation Examples 11 to 16, L-valine, L-isoleucine, L-leucine, citric acid were dissolved in warm water, and the solution was concentrated to crystallize by a vacuum evaporator. Solid was separated from the concentrated solution, and the solid was dried. A solid solution having a weight ratio of valine:leucine:isoleucine:citric acid being 23:45:23:9 was obtained.

[Preparation Example 22]

Similar to Preparation Examples 11 to 16, L-valine, L-isoleucine, L-leucine, L-asparagine hydrate were dissolved in warm water, and the solution was concentrated to crystallize by a vacuum evaporator. Solid was separated from the concentrated solution, and the solid was dried. A solid solution having a weight ratio of valine:leucine:isoleucine:asparagines hydrate being 20:43:20:17 was obtained.

[Preparation Example 23]

Similar to Preparation Examples 11 to 16, L-valine, L-isoleucine, L-leucine, L-aspartic acid were dissolved in warm water, and the solution was concentrated to crystallize by a vacuum evaporator. Solid was separated from the concentrated solution, and the solid was dried. A solid solution having a weight ratio of valine:leucine:isoleucine:aspartic acid being 15:44:18:23 was obtained.

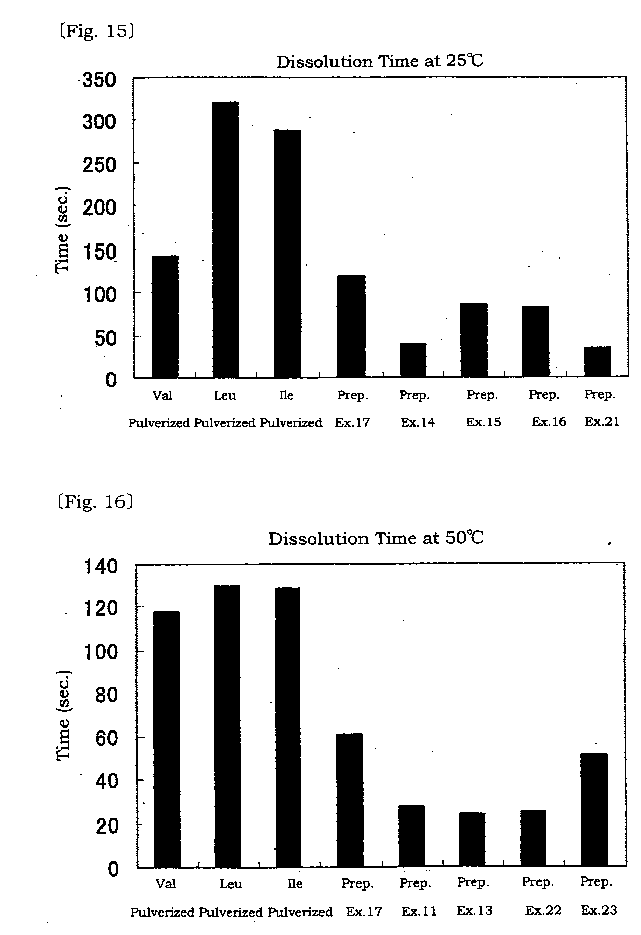

As to the composition shown in Table 4 and Table 5, a mean particle size and a dissolution speed at ordinary temperature and at 50 degrees were examined. The dissolution speed of each sample was shown in figure 15, Figure 16. From the figures, it was found that, even considering the dispersion of the mean particle sizes of various samples, the dissolution speed of the solid solutions is still remarkably fast.

[Table 4]

| 25 degrees Sample | | | |
|---|---|---|---|
| Sample Name | Amino Acid Composition (ratio by weight) | Mean Particle Size | Dissolution Speed |
| Val Pulverized | Val =100 w/w % | 210 μm | 142 sec. |
| Leu Pulverized | Leu =100 w/w % | 280 μm | 320 sec. |
| Ile Pulverized | Ile =100 w/w % | 400 μm | 287 sec. |
| Prep. Ex. 17 | Val:Leu:Ile=12:62:25 | 230 μm | 117.5 sec. |
| Prep. Ex. 14 | Val:Leu:Ile:malic acid=20:40:20:20 | 950 μm | 39 sec. |
| Prep. Ex. 15 | Val:Leu:Ile:Gly=23:51:26:0.4 | 250 μm | 86 sec. |
| Prep. Ex. 16 | Val:Leu:Ile:Gln=24:50:25:0.3 | 320 μm | 81.5 sec. |
| Prep. Ex. 21 | Val:Leu:Ile:citric acid=23:45:23:9 | 720 μm | 34 sec. |

[Table 5]

| 50 degrees Sample | | | |
|---|---|---|---|
| Sample Name | Amino Acid Composition (ratio by weight) | Mean Particle Size | Dissolution Speed |
| Val Pulverized | Val =100 w/w % | 210 μm | 117.5 sec. |
| Leu Pulverized | Leu =100 w/w % | 280 μm | 130 sec. |
| Ile Pulverized | Ile =100 w/w % | 400 μm | 128.5 sec. |
| Prep. Ex. 17 | Val:Leu:Ile=12:62:25 | 230 μm | 61 sec. |
| Prep. Ex. 11 | Val:Leu:Ile:Thr=15:51:23:11 | 270 μm | 27.5 sec. |
| Prep. Ex. 13 | Val:Leu:Ile:Glu=17:46:20:17 | 210 μm | 24 sec. |
| Prep. Ex. 22 | Val:Leu:Ile:Asn-H2O=20:43:20:17 | 230 μm | 25 sec. |
| Prep. Ex. 23 | Val:Leu:Ile:Asp=15:44:18:23 | 230 μm | 51.5 sec. |

Main peaks and peak strength of threonine, glutamic acid, malic acid and peak strength of the solid solutions of Preparation Examples 2 to 4, Comparative Examples 2 to 4 in the powder X-ray diffraction spectrum are shown in Table 6.

[Table 6]

| Sample Name | Peak No. | Diffraction Angle (2θ) | Relative strength (strongest peak is designated 100) |
|---|---|---|---|
| Threonine | Fig. 10-1 | 34.9988 | 100.00 |
| | Fig. 10-2 | 28.5125 | 77.49 |
| Prep. Ex. 12 | Fig. 10-1 | Not detected | 0.00 |
| | Fig. 10-2 | Not detected | 0.00 |
| Prep. Ex. 18 | Fig. 10-1 | 35.0635 | 1.88 |
| | Fig. 10-2 | 28.5871 | 1.20 |
| Glutamic Acid | Fig. 11-3 | 20.5443 | 100.00 |

(continued)

| Sample Name | Peak No. | Diffraction Angle (2θ) | Relative strength (strongest peak is designated 100) |
|---|---|---|---|
| | Fig. 11-4 | 10.2527 | 73.08 |
| Prep. Ex. 13 | Fig. 11-3 | Not detected | 0.00 |
| | Fig. 11-4 | Not detected | 0.00 |
| Prep. Ex. 19 | Fig. 11-3 | 20.5376 | 5.08 |
| | Fig. 11-4 | 10.2464 | 3.38 |
| Malic acid | Fig. 12-5 | 20.0982 | 100.00 |
| | Fig. 12-6 | 23.5427 | 40.90 |
| Prep. Ex. 14 | Fig. 12-5 | Not detected | 0.00 |
| | Fig. 12-6 | Not detected | 0.00 |
| Prep. Ex. 20 | Fig. 12-5 | 20.1019 | 13.11 |
| | Fig. 12-6 | 23.5397 | 5.57 |

Industrial Applicability

The solid solution of the invention can vary the ratio of valine, isoleucine, leucine widely, and renders bitter taste less and raises dissolution speed. Therefore, it can be utilized widely for foods, medical ones, containing valine, isoleucine, leucine.

**Claims**

1. A solid solution composed of three amino acids of valine, isoleucine, leucine.

2. A solid solution as set forth in claim 1, having a composition ratio of 5-85 w/w % of valine, 3-80 w/w % of isoleucine, 3-80 w/w % of leucine.

3. A solid solution as set forth in claim 1 or 2, wherein a powder X-ray diffraction pattern indicates main peaks at a diffraction angle (2θ) of 6-7, 12-14 (unit:degree).

4. A solid solution composed of valine, isoleucine, leucine and at least one of acid selected from the group consisting of neutral aliphatic amino acid, neutral hydroxy amino acid, neutral acid amide amino acid, acidic amino acid, basic amino acid, hydroxy acid.

5. A solid solution as set forth in claim 4, wherein the aliphatic amino acid is glycine, alanine, norleucine, homoleucine.

6. A solid solution as set forth in claim 4, wherein the neutral hydroxy amino acid is serine, threonine.

7. A solic solution as set forth in claim 4, wherein the neutral acid amide amino acid is asparagine monohydrate, glutamine.

8. A solid solution as set forth in claim 4, wherein the acidic amino acid is glutamic acid, asparatic acid.

9. A solid solution as set forth in claim 4, wherein the basic amino acid is arginine, lysine, histidine.

10. A solid solution as set forth in claim 4, wherein the hydroxy acid is melic acid, citric acid, tartaric acid.

11. A solid solution as set forth in any one of claims 5-10, which has a composition ratio of 5-85 w/w % of valine, 3-80 w/w % of isoleucine, 3-80 w/w % of leucine, 0.01-50 w/w % of at least one of acid selected from the group consisting of neutral aliphatic amino acid, neutral hydroxy amino acid, neutral acid amide amino acid, acidic amino acid, basic amino acid, hydroxy acid.

12. A solid solution as set forth in any one of claims 5-11, wherein a powder X-ray diffraction pattern indicates the strongest peak at a diffraction angle (2θ) of 6-7°, and the first, second strongest peaks existing at the position shown below derived from at least one of acid selected from the group consisting of neutral aliphatic amino acid, neutral hydroxy amino acid, neutral acid amide amino acid, acidic amino acid, basic amino acid, hydroxy acid, are not detected, or detected as smaller peaks than those of a powder mixture having the same composition:

Glycine diffraction angle (2θ) = 29.8°, 23.9°, alanine diffraction angle (2θ) = 20.6°, 28.9°, threonine diffraction angle (2θ) = 35.0°, 28.5°, glutamine diffraction angle (2θ) = 23.4° ,25.0°, asparagines monohydrate diffraction angle (2θ) = 29.2°, 18.1°, glutamic acid diffraction angle (2θ) = 10.3° ,20.5°, aspartic acid diffraction angle (2θ) = 23.8° ,11.9°, arginine diffraction angle (2θ) = 18.3°, 27.4°, citric acid diffraction angle (2θ) = 15.2°, 18.2°, malic acid diffraction angle (2θ) = 20.1°, 23.5°, tartaric acid diffraction angle (2θ) = 20.7°, 35.8°.

13. A solid solution as set forth in any one of claims 1-12, having a mean particle size of 50-1000 μm.

14. A solid medicine or a solid food produced using a solid solution as set forth in any one of claims 1-13.

15. A food containing the amino acid composition as set forth in claim 11.

16. A method of manufacturing a solid solution composed of three amino acids of valine, isoleucine, leucine, which comprises concentrating an aqueous solution of 0.3-10.0 w/w % of valine, 0.3-6.5 w/w % of isoleucine, 0.3-4.0 w/w % of leucine at 40-90°C under a pressure of 3 kPa-30 kPa to crystallize, cooling the concentrated solution to 15-25°C, and then separating crystals.

17. A manufacture method as set forth in claim 16, wherein, designating the total amount of isoleucine and leucine in the raw aqueous solution A [g], the weight of valine B [g], the weight ratio of valine in the produced solid solution V [w %], the effective distribution coefficient of valine against the total amount of isoleucine and leucine f [-], the crystallization yield of isoleucine and leucine in the produced solid solution Y [w %].
The content of valine is controlled in the solid solution (composed of 5-85 w % of valine, 3-80 w % of isoleucine, 3-80 w % of leucine) based on the relationship;

$$V = 100fB/\{fB + A \, (100 - Y + fY) \, / \, 100\} \cdots 1$$

Wherein, designating the total amount of isoleucine and leucine in the raw aqueous solution A [g], the weight of valine B [g], the weight ratio of valine in the produced solid solution V [w %], the effective distribution coefficient of valine against the total amount of isoleucine and leucine f [-], the crystallization yield of isoleucine and leucine in the produced solid solution Y [w %].

18. A manufacture method as set forth in claim 16, wherein, the effective distribution coefficient f [-] as set forth in claim 17 is calculated by the formula;
when the weight ratio of isoleucine to leucine is the produced solid solution is 2 or less,

$$0.226 \, x \, \, [\text{Val}/(\text{Ile} + \text{Leu})]_{\text{solid solution}} + 0.078 \cdots 2$$

when the weight ratio exceeds 2,

$$0.504 \, x \, \, [\text{Val}/(\text{Ile} + \text{Leu})]_{\text{solid solution}} + 0.029 \cdots 3$$

Herein, $[\text{Val}/(\text{Ile} + \text{Leu})]_{\text{solid solution}}$ represents weight ratio [w %] of valine to the total amount of isoleucine and leucine in the solid solution.

19. A method of producing a solid solution containing valine, isoleucine, leucine and at least one of acid selected from the group consisting of neutral aliphatic amino acid, neutral hydroxy amino acid, neutral acid amide amino acid, acidic amino acid, basic amino acid, hydroxy acid, which comprises crystallizing using a solution containing valine, isoleucine, leucine and at least one of acid selected from the group consisting of neutral aliphatic amino acid, neutral hydroxy amino acid, neutral acid amide amino acid, acidic amino acid, basic amino acid, hydroxy acid.

EP 2 351 562 A1

[Fig. 1]

(1) Val
(2) Ile
(3) Leu
(4) Val:Leu:Ile=25:50:25

BCAA-SS080207-1.xrdml

Counts

Position [° 2Theta]

〔Fig. 2〕

[Fig. 3]

[Fig. 4]

[Fig. 5]

[Fig. 6]

Dissolution speed of various samples

[Fig. 7]

Results of sensory evaluation of solid solution
against mixture

[Fig. 8]

[Fig. 9]

[Fig. 10]

A : Preparation Example 17   B : Preparation Example 11   C : Preparation Example 12

D : Preparation Example 18   E : Threonine

[Fig. 11]

A : Preparation Example 17   B : Preparation Example 13   C : Preparation Example 19
D : Glutamic acid

[Fig. 12]

A : Preparation Example 17   B : Preparation Example 14   C : Preparation Example 20
D : Malic acid

[Fig. 13]

A : Preparation Example 17     B : Preparation Example 15     C : Glycine

[Fig. 14]

A : Preparation Example 17    B : Preparation Example 16    C : Glutamine

[Fig. 15]

Dissolution Time at 25℃

[Fig. 16]

Dissolution Time at 50℃

## INTERNATIONAL SEARCH REPORT

International application No.

PCT/JP2009/005626

### A. CLASSIFICATION OF SUBJECT MATTER

*A61K31/198*(2006.01)i, *A61K9/14*(2006.01)i, *A61P3/00*(2006.01)i, *A61P3/02*
(2006.01)i, *A61P7/00*(2006.01)i, *A61P21/00*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K31/198, A61K9/14, A61P3/00, A61P3/02, A61P7/00, A61P21/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/BIOSIS/MEDLINE/WPIDS(STN), JSTPlus/JMEDPlus/JST7580(JDreamII)

### C. DOCUMENTS CONSIDERED TO BE RELEVANT

| Category* | Citation of document, with indication, where appropriate, of the relevant passages | Relevant to claim No. |
|---|---|---|
| A | KUROSAWA, I. et al, Ind. Eng. Chem. Res., 2005, Vol.44, pp.3284-3288 | 1-19 |
| A | KUROSAWA, I et al, Fluid Phase Equilibria, 2004, Vol.224, pp.245-249 | 1-19 |
| A | GANAPATHY, S. et al, Indian Journal of Biochemistry & Biophysics, 1977, Vol.14, pp.211-213 | 1-19 |
| A | JP 55-099164 A (Central Institute for Experimental Animals), 27 July 1980 (27.07.1980), entire text (Family: none) | 1-19 |

☐ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 November, 2009 (13.11.09) | 24 November, 2009 (24.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Kogyo Chosa kai.** Amino Acid Handbook. Ajinomoto Co., Inc, 01 April 2003, 47
- *Ind. Eng. Chem. Res.,* 2005, vol. 44, 3284-3288
- *Fluid Phase Equilibria,* 2004, vol. 224, 245-249